# EUROPEAN PATENT APPLICATION

(11) **EP 2 777 504 A1**
(43) Date of publication of application: **17.09.2014**
(21) Application number: 14425026.3
(22) Date of filing: 12.03.2014
(51) Int. Cl.: A61B 6/00, G01N 23/04, G01V 5/00, H05H 7/00

(54) **Multi-angle industrial radiography and radioscopy apparatus**

(30) Priority: 12.03.2013 IT RM20130145
(71) Applicant: Gatto, Pompilio, 00118 Roma (RM) (IT); Nicoletti, Gaetano, 07040 Stintino (SS) (IT)
(72) Inventor: Gatto, Pompilio, 00118 Roma (RM) (IT); Nicoletti, Gaetano, 07040 Stintino (SS) (IT)

(57) **Abstract**

Apparatus for emitting X-rays for radiography and radioscopy, comprising an arm (1), supported slideably in a vertical direction by a stand (11), including an X-ray radiating head (5) comprising a linear electron accelerator or linac (7) supplied with radiofrequency electromagnetic waves from an electromagnetic wave generating assembly (3) on the arm (1) through an electromagnetic waveguide transmission line including a dual rotating coupling including two rotating couplings (21; 23), each one having a respective fixed and a respective mobile, rotating portion, mechanically series-connected perpendicularly to each other, able to preserve the electrical continuity along said transmission line means; a first, roll rotating coupling (21) thereof being assembled integral with said arm (1), whilst a second, pitch rotating coupling (23) is assembled integral with said radiating head (5), so that the radiating head (5) through the rotating couplings (21; 23) is capable of being oriented multi-angularly with respect to a piece under test; and kit of such an apparatus with a diode array X-ray detector in functional communication with a computer, to transmit digital image signals thereto.

## Description

The present invention relates to an industrial radiography and radioscopy apparatus.

As according to general usage, the term 'radiography' is to be construed in the present application as an operation of visually inspecting the internal status of a piece, meant in the broadest sense, in regard to internal cracks, such as for instance to ascertain the internal status of the bronze constituting a statue of ancient art, to see if it has undergone corrosion, for restoration purposes.

As opposed, the term 'radioscopy' or 'videoscopy' is to be construed in the present application as an operation again of visually inspecting a piece to check what the piece includes in its interior, particularly for detecting hidden objects; by 'piece' particularly fixed containing means and mobile containing means (mobile containers; lorries; TIRs and transportation means in general and/or packing means of any type) being meant.

Radiography and radioscopy are implemented by exploiting the property of ionizing radiation, particularly X and gamma rays, to penetrate various materials to visually inspect objects for features that cannot be viewed directly. In radiography and radioscopy a piece is scanned with ionizing radiation, and an image is taken by making the irradiation past the piece to impinge on an X-ray detector imaging means which render a visual image which is directly interpretable by man, such as radiographic imaging plates.

Industrial radiography is mainly aimed at detecting hidden flaws of materials (non-destructive testing), including metal and non-metal (e.g. ceramic) objects.

Industrial radiography is particularly used for weld and joint inspection and grading, for instance in pressurized piping, pressure vessels, high-capacity storage containers; for locating reinforcing bars and conduits in concrete, for locating anomalies in parts caused by corrosion or mechanical damage, where it is necessary to have an absolute certainty of a perfect status of a component or part under test, up to the issue of a certified documentation including radiographic digital imaging plates or copies.

Videoscopy is regularly used in airports to examine luggage, using X-ray radiography, and at the customs to scan cargo containers and articulated lorries using X-ray or gamma-ray radiography.

Apparatuses for performing industrial radiography and radioscopy are based on linear electron accelerators. A linear electron accelerator, or shortly linac, is an evacuated waveguide wherein a high-frequency travelling electromagnetic wave is excited, the axial electric field whereof accelerates electrons. The phase velocity of the wave is made equal to the velocity of the electrons by assembling irises in the waveguide, that delimit accelerating coupled resonating cavities. The linear accelerator is fed by a radiofrequency oscillator, such as a cavity magnetron, through an electromagnetic wave guiding structure, such as a waveguide or a coaxial cable. A feature of a linear accelerator is the beam collimation thereof, i.e. the parallelism of the trajectories of the electrons it emits.

An industrial radiographic apparatus of the state-of-the-art includes a mobile radiating head including a linac therein; a radiofrequency generator assembly including a radiofrequency oscillator therein; a stand, assembled on a mobile motor-equipped baseplate, supporting the radiating head by means of an arm through a coupling that allows a roll and a pitch of the radiating head; a control cabinet including hardware for controlling the movements of the apparatus, separate from the apparatus; and a control console.

In the radiography and radioscopy apparatuses of the state-of-the-art the radiofrequency generator assembly is arranged in a box, separate from the radiating head, beyond the coupling of the latter with the arm. The waveguide connecting the linac to the radiofrequency generator assembly is a flexible waveguide which steps over the coupling, outside the apparatus.

A number of shortcomings are associated with the apparatuses of the state-of-the-art.

A main shortcoming is that said flexible connection waveguide is a cumbersome member; it undergoes bending and twisting strains in the rotation of the radiating head during the operation of the apparatus, whereby it is subjected to wear, up to rupture. Moreover, it requires accurate tuning settings to guarantee the signal integrity therethrough to preserve the radiofrequency supplied to the linac. Finally, and very importantly, it imposes a restriction of angular range of the pitch of the radiating head within ±45°. This involves the restriction that a front static scanning of objects is only allowed with radiography and radioscopy apparatues of the state-of-the-art.

It is the main object of the present invention to remove such a shortcoming.

According to the present invention, such an object is reached with the novel contrivance of making the electromagnetic waveguide means electrically connecting the linac to the radiofrequency electromagnetic wave generating assembly through a waveguide transmission line comprising a dual rotating coupling including two rotating couplings, each one having a respective fixed and a respective mobile, rotating portion, mechanically series-connected perpendicularly to each other, able to preserve the electrical continuity along the transmission line; the first, roll rotating coupling thereof being assembled integrally with the arm, whilst the second, pitch rotating coupling is assembled integrally with the radiating head.

By virtue of the novel contrivance of such a dual rotating coupling, the radiating head of the apparatus is capable of being oriented multi-angularly with respect to a piece under test. So, the radiating head of the inventive apparatus can be put in an optimum aiming position to activate a radiography or radioscopy task from a variety of angle shots and viewpoints in space, allowing to perform scans on a piece without having to move neither the piece, nor the apparatus.

Moreover, again by virtue of the dual rotating coupling, it is possible to make the waveguide means rigid, particularly ferrous rigid material, which better guarantee signal integrity therethrough.

According to the present invention, the bedplate is equipped with motor means and with actuating wheels driven thereby independently of each other and capable of revolving through ±180° on the plane.

Another drawback of the apparatuses of state-of-the-art is that the control unit thereof is a unit physically separated from the apparatus itself, which reduces the mobility of the apparatus, and is a problem for the use of the apparatus in impervious places.

It is another object of the present invention to provide a compact radiography and radioscopy apparatus, so overcoming such a drawback.

So, the present invention envisages that the cooling means and the control means of the apparatus are arranged on the baseplate of the apparatus.

Another object of the present invention is to provide a radiography and radioscopy apparatus which turns out to be safe for the operator, in consideration of the danger of the X rays emitted by the apparatus itself, and of the fact that the pieces to be tested themselves may be dangerous, as their content is not surely known before a scanning thereof.

Therefore, according to a preferred embodiment, the control means are equipped with wireless link means for receiving remote controls, and further include a remote control unit for emitting controls thereto. So the apparatus can be controlled remotely, for the sake of safety of the operator, and the aforesaid object is reached. Indeed, the operator is able to remotely control the apparatus by transmitting controls thereto and receiving remote data signals through a wireless link. Such a feature gives the possibility of using the inventive apparatus from a point at such a distance as to ensure the safety of the operator.

The apparatus of the present invention is to be used in cooperation with X-ray detector means for imaging thereon at each irradiation with X rays.

In this connection, another object of the present invention is to exploit computer means for storing and processing the radiographic and radioscopic images taken with the apparatus of the present invention.

Such an object is reached by providing the X-ray detector means as a diode array, endowed with means for converting images, acquired upon irradiation with X rays, into digital image signals, and in functional communication with digital automatic programmable computer means, to transmit the digital image signals thereto; the computer means being so programmed as to enable reconstruction, storing and processing of the digital images it receives from the diode array.

The diode array is to be put on the ground and is capable of taking very detailed images and (dynamic) videos; it enables storing, particularly in a database, and processing with a computer, as opposed to usual radiographic plates. In the case of videoscopy, videos may be viewed subsequently, particularly at various speeds, and for viewing selected freeze-frames rousing attention.

Under another aspect, the present invention takes its steps from considering that the knowledge is useful of the angular position of the radiating head in space (with respect to a reference system integral to the apparatus), which also depends on the (vector) product of roll and pitch rotations thereof in the operation of the apparatus, for the purposes of the docking manoeuvre and the right positioning of a member.

Said knowledge is also useful for the purpose of remotely checking the well-operating status of the apparatus, which then can be tested by verifying the right response thereof to controls.

Therefore, the present invention envisages that the apparatus further includes sensor means for sensing the respective roll and pitch angular position values of the mobile portions of the rotating couplings with respect to the respective fixed portions thereof; and angular position computing means, in functional communication with the sensor means, capable of computing the spatial angular position of the X-ray beam; the spatial angular position of the radiating head, taking the roll and pitch angular positions, necessary for such a computing, into account; the angular position computing means being in functional communication with radiating head angular position control means.

Designing such computing means falls within the average skill in the art and so they are not disclosed herein.

The sensor means may be embodied by encoders.

Therefore, it is the subject-matter of the present invention an apparatus for emitting X-rays for radiography and radioscopy according to annexed Claim 1.

It is also the subject-matter of the present invention a kit of an apparatus for emitting X-rays for radiography and radioscopy and of an X-ray detector means for imaging thereon at each irradiation with X rays according to annexed Claim 6.

It is also the subject-matter of the present invention an apparatus for emitting X-rays for radiography and radioscopy according to annexed Claim 8.

Preferred embodiments are set forth in annexed Claims 2; 3; 4; 5; 7; 9; 10.

The mechanics of the inventive apparatus allows it to be used in a variety of places, allowing a handy and quick logistics therefor, as it is portable, compact and easily handled; it may be used with ease in both suitably set up rooms, such as bunkers or industrial sheds and in the open air, such as in yards.

The same inventive apparatus may be used for two specific functions:
*(a)* the execution of industrial radiography, in non-destructive tests, providing a qualitative check whereby possible macro- or microdefects are put into evidence without destroying or damaging the piece under test;
(b) the execution of radioscopic inspection i.e. videoscopy, in particular of containing means such as: containers; TIRs; transportation means in general and/or packings of any nature.

The present invention is disclosed in the following having reference to preferred embodiments thereof, only given as a matter of example, absolutely not of restriction of the original teaching thereof, wherein:
- FIGURE 1 depicts an apparatus of the present invention in partially cut-away side view;
- FIGURE 2 depicts a side view of an X-ray detector diode array for use with the apparatus for performing a radiography or radioscopy task.

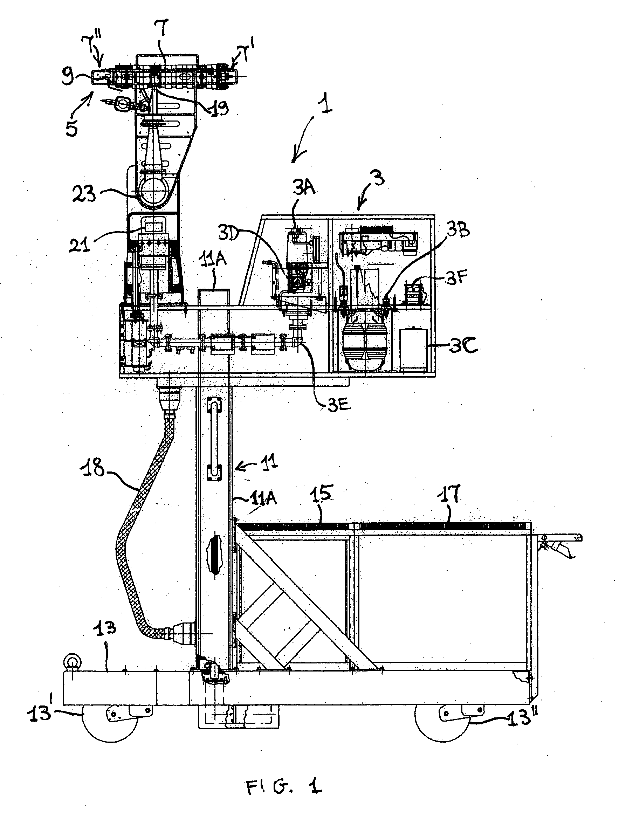

Referring to FIGURE 1, the present invention is an apparatus for emitting X-rays for radiography and radioscopy, comprising an arm **1** including a radiofrequency electromagnetic wave generating assembly **3** and an X-ray radiating head **5**. Radiofrequency generating assembly **3** includes oscillator means, such as magnetron or klystron **3A** and thyratron **3B;** a pulse shaping line **3C;** a circulator **3D;** output electromagnetic waveguide means **3E,** and interface electronic boards **3F.** Radiating head **5** comprises a linear electron accelerator or shortly linac **7** endowed with an electron gun constituting a cathode at a cathode extremity **7'** thereof, and, at an opposite, X-ray output extremity **7"** thereof, endowed with a target **9** capable of converting the electron beam emitted by said cathode into an X-ray beam constituting a characteristic X-radiation useful for radiographies and radioscopies, together with *Brehmsstrahlung* radiation. Target **9** may be a tungsten target.

The mounting structure of the apparatus is a stand **11** comprising a bedplate **13** and an upright **11A** integral thereto, bearing arm **1** slideably in the vertical direction. The apparatus is equipped with a cooling assembly **15** for keeping the apparatus at a right working temperature; and with a control apparatus **17** for controlling the operation of the apparatus, both through a cable **18** connected with arm **1.**

Linac **7** is supplied with radiofrequency electromagnetic waves by radiofrequency electromagnetic wave generating assembly **3** through electromagnetic waveguide means **3E.** Linac **7** is endowed with a vacuum-tight window **19,** laterally thereon, at a position between said cathode extremity **7'** and said output extremity **7"** of linac **7,** which window is transparent to electromagnetic waves, allowing the transit of the radiofrequency electromagnetic waves into the interior of linac **7,** whilst keeping a vacuum inside it. Two ion pumps provide for keeping the right vacuum level inside linac **7.** Vacuum-tight window **19** may be a ceramic window.

Electromagnetic waveguide means **3E** constitute a waveguide transmission line electromagnetically connecting linac **7** to radiofrequency electromagnetic wave generating assembly **3** and preceptively comprise a dual rotating coupling including two rotating couplings **21; 23,** each one having a respective fixed and a respective mobile, rotating portion, mechanically series-connected perpendicularly to each other, able to preserve the electrical continuity along said transmission line; the first, roll rotating coupling **21** thereof being assembled integrally with arm **1,** whilst the second, pitch rotating coupling **23** is assembled integrally with radiating head **5.** So radiating head **5** is capable, through rotating couplings **21; 23,** of being oriented, in a novel way, multi-angularly with respect to a piece under test.

According to a preferred embodiment the electromagnetic waveguide means are rigid waveguides, particularly of a ferrous material.

According to a preferred embodiment, bedplate **13** is equipped with motor means and with actuating wheels **13'; 13",** driven thereby independently of each other and capable of revolving through ±180° on the plane.

According to a preferred embodiment, cooling assembly **15** and control apparatus **17** are directly arranged on baseplate **13.**

According to an embodiment, control apparatus **17** is equipped with wireless link means, such as for instance *wi*fi, for receiving remote controls, and further includes a remote control unit for emitting controls thereto, so that control apparatus **17** may be controlled remotely.

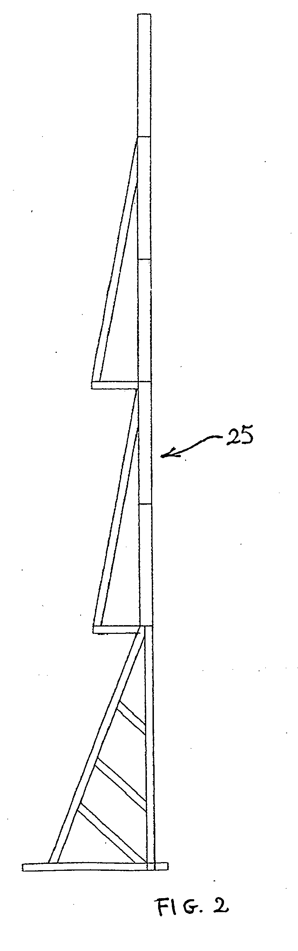

Referring to FIGURE 2, the present invention is also a kit of an apparatus as disclosed above and of an X-ray detector diode array **25,** endowed with means for converting images, acquired upon irradiation with X rays, into digital image signals, and in functional communication with digital automatic programmable computer means, to transmit the digital image signals thereto. The computer means are so programmed as to enable reconstruction, storing and processing of the digital images they receive from the diode array. It is particularly envisaged that the diode array is in functional communication with the computer means through wireless link means, such as *wi-fi.*

Under another aspect, the present invention is an apparatus as disclosed above, further including sensor means for sensing the respective roll and pitch angular position values of the mobile portions of rotating couplings **21; 23** with respect to the respective fixed portions thereof; and angular position computing means, in functional communication with said sensor means, capable of computing the spatial angular position of said X-ray beam, i.e. the spatial angular position of radiating head **5,** taking the roll and pitch angular positions, necessary for such a computing, into account. The angular position computing means are in functional communication with radiating head angular position control means.

Particularly, the computing means may be in functional communication with means for displaying the angular position values to a human operator, who will consequently perform the right intervention to keep the right position.

As an alternative, the control apparatus further is equipped with automatic control means for keeping radiating head **5** in its right position, and said computing means are in functional communication therewith to provide them with the actual spatial angular position of radiating head **5** to enable them to actuate the right correction action automatically, without an operator intervention.

Embodiments of the inventive apparatus provide an electron beam with energy of 5 MeV or 3 MeV.

With an inventive apparatus it is possible to carry out a radioscopy of a lorry having a length of 18 metres within about a minute.

The versatility of the inventive apparatus allows the installation thereof both inside a bunker and on a lorry trailer. It may also be installed in a hypogeal bunker, so as to cut the first-plant costs off.

The present invention has been disclosed and illustrated having reference to specific embodiments thereof, but it is to be understood that variations, additions or omissions may be made without so departing from the scope of protection thereof, which only remains restricted by the appended claims.

## Claims

1. An apparatus for emitting X-rays for radiography and radioscopy, comprising, in combination:
an arm (1) including a radiofrequency electromagnetic wave generating assembly (3) and an X-ray radiating head (5) comprising a linear electron accelerator or linac (7) endowed with an electron gun constituting a cathode at a cathode extremity thereof (7'), and, at an opposite, output extremity thereof (7"), endowed with a target (9), capable of converting the electron beam emitted by said cathode into an X-ray beam;
a stand (11) comprising a bedplate (13), and an upright (11A) integral thereto, bearing said arm (1) slideably in a vertical direction;
cooling means (15) for keeping the apparatus at a right working temperature;
control means (17) for controlling the operation of the apparatus;
said cooling means (15) and said control means (17) being functionally connected to said arm (1) through cable means (18);
said linac (7) being supplied with radiofrequency electromagnetic waves by said radiofrequency electromagnetic wave generating assembly (3) through electromagnetic waveguide means (3E);
said linac (7) being endowed with a vacuum-tight window (19), at a position laterally between said linac cathode extremity (7') and said linac output extremity (7"), transparent to electromagnetic waves, allowing the transit of said radiofrequency electromagnetic waves into the interior of said linac (7), whilst keeping a vacuum inside it;
said electromagnetic waveguide means (3E) electrically connecting said linac (7) to said radiofrequency electromagnetic wave generating assembly (3) through waveguide transmission line means including a dual rotating coupling including two rotating couplings (21; 23), each one having a respective fixed and a respective mobile, rotating portion, mechanically series-connected perpendicularly to each other, able to preserve the electrical continuity along said transmission line means; a first, roll rotating coupling (21) thereof being assembled integral with said arm (1), whilst a second, pitch rotating coupling (23) is assembled integral with said radiating head (5),
so that said radiating head (5) through said rotating couplings (21; 23) is capable of being oriented multi-angularly with respect to a piece under test.

2. The apparatus of Claim 1, wherein said electromagnetic waveguide means are rigid waveguides.

3. The apparatus of Claim 1, wherein said bedplate (13) is equipped with motor means and with actuating wheels (13'; 13"), driven thereby independently of each other and capable of revolving through ±180° on the plane.

4. The apparatus of Claim 1, wherein said cooling means (15) and said control means (17) are directly arranged on said baseplate (13), for the sake of compactness of the apparatus.

5. Apparatus of Claim 1, wherein said control means (17) are equipped with wireless link means for receiving remote controls, and further include a remote control unit for emitting controls thereto, so that the apparatus can be controlled remotely, for the sake of safety of the operator.

6. A kit of an apparatus according to Claim 1 and of a diode array X-ray detector means (25), endowed with means for converting images, acquired upon irradiation with X rays, into digital image signals, and in functional communication with digital automatic programmable computer means, to transmit said digital image signals thereto; said computer means being so programmed as to enable reconstruction, storing and processing of said digital images it receives from said diode array.

7. The kit of Claim 6, wherein said diode array detector means (25) are in functional communication with said computer means through wireless link means.

8. The apparatus of Claim 1, further including sensor means for sensing the respective roll and pitch angular position values of said mobile portions of said rotating couplings (21; 23) with respect to the respective fixed portions thereof; and angular position computing means, in functional communication with said sensor means capable of computing the spatial angular position of said X-ray beam; the spatial angular position of said radiating head, taking said roll and pitch angular positions, necessary for such a computing, into account; said angular position computing means being in functional communication with radiating head angular position control means.

9. The apparatus of Claim 8, wherein said computing means are in functional communication with means for displaying said angular position values to a human operator.

10. The apparatus of Claim 8, wherein said control means are further equipped with automatic control means for keeping said radiating head (5) in its right position, and said angular position computing means are in functional communication therewith to provide them with the actual spatial angular position of said radiating head (5) to enable them to actuate the right correction action.
